# FASCICULE DE BREVET EUROPEEN

(11) **EP 1 814 998 B1**
(45) Date de publication et mention de la délivrance du brevet: **03.08.2011**
(21) Numéro de dépôt: 05801663.5
(22) Date de dépôt: 08.11.2005
(51) Int. Cl.: C12N 15/85, C12N 15/90, A01K 67/027

(54) **ANIMAUX MODELES COMPRENANT AU MOINS UN KO ET UN KI CONDITIONNEL**
TIERMODELLE MIT MINDESTENS EINEM KONDITIONALEN KO UND EINEM KONDITIONALEN KI
ANIMAL MODELS COMPRISING AT LEAST ONE CONDITIONAL KO AND ONE CONDITIONAL KI

(30) Priorité: 08.11.2004 FR 0411875
(43) Date de publication de la demande: 08.08.2007
(73) Titulaire: Genoway, 69007 Lyon (FR)
(72) Inventeur: THIAM, Kader, F-69600 Oullins (FR); FRAICHARD, Alexandre, F-69007 LYON (FR)
(74) Mandataire: Tetaz, Franck Claude Edouard
(86) Numéro de dépôt international: PCT/EP2005/055828
(87) Numéro de publication internationale: WO 2006/048466

(56) Documents cités:
- WO-A-02/40685
- US-A1- 2003 165 497
- US-A1- 2004 241 851
- KUEHN R ET AL: "CONDITIONAL KNOCKOUT MICE" 2003, METHODS IN MOLECULAR BIOLOGY, HUMANA PRESS INC., CLIFTON, NJ, US, PAGE(S) 159-185 , XP001206819 cité dans la demande page 166 - page 168; figure 4
- MCMINN, JULIE E. ET AL: "An allelic series for the leptin receptor gene generated by CRE and FLP recombinase" MAMMALIAN GENOME, vol. 15, no. 9, septembre 2004 (2004-09), pages 677-685, XP002332118 ISSN: 0938-8990 cité dans la demande
- LIU PENTAO ET AL: "A highly efficient recombineering-based method for generating conditional knockout mutations." GENOME RESEARCH, vol. 13, no. 3, mars 2003 (2003-03), pages 476-484, XP002376686 ISSN: 1088-9051
- TIAN E ET AL: "Otx2 is required to respond to signals from anterior neural ridge for forebrain specification" DEVELOPMENTAL BIOLOGY, vol. 242, no. 2, 15 février 2002 (2002-02-15), pages 204-223, XP002376687 ISSN: 0012-1606
- EMAMBOKUS NIKLA R ET AL: "A c-myb mutant allelic series generated by Cre- and Flp-mediated recombination reveals different requirements for the c-myb transcription factor in myeloid progenitors" BLOOD, vol. 98, no. 11 Part 1, 16 novembre 2001 (2001-11-16), page 793a, XP009065164 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971

## Description

La présente invention concerne un vecteur permettant de développer des animaux modèles comprenant d'une part un KO (Knock Out) dans un gène cible et d'autre part une modification de son génome afin d'introduire un nouveau gène (KI ou Knock In) pour être exprimé de manière conditionnelle et des éléments permettant de restaurer le locus endogène où le vecteur a été intégré. La présente invention concerne également les cellules eucaryotes transformée avec le vecteur selon l'invention, et les embryons et animaux génétiquement modifiés comprenant les cellules selon l'invention.

Le vecteur selon l'invention permet de réaliser sur le même modèle animal plusieurs opérations nécessitant autrefois le développement et l'usage de plusieurs modèles distincts. Il permet par exemple, d'étudier avec le même modèle le phénotype obtenu par le KO d'un gène cible, puis l'expression d'un transgène donné en place de ce gène cible,et le cas échéant l'inactivation du transgène.

En outre, ces phénotypes peuvent être observés de manière tissus-spécifiques, par exemple en obtenant une expression du transgène ou la restauration du phénotype uniquement dans certains tissus.

Différentes stratégies à base de recombinases et de vecteurs permettant d'insérer puis de supprimer des éléments de gènes dans les génomes de différents organismes sont décrits dans l'état de la technique, notamment dans EP 1 462 525, US 2003/165497, US 2004/241851, WO 02/40685, WO 2004/056964, WO 2004/044151, WO 2005/007877, Kasin & al (Nucleic Acid Research. 2004, vol 32, n° 7, pp E66.1-E66.8), Coumoul & al (Nucleic Acid Research 2004, vol 32, n° 10, pp E85.1-E85.7), Conrad & al. (Biotechnologies, vol 34, n° 6, juin 2003, 1136-1140), Kühn & al. (Methods in Molecular Biology 2003, vol 209, 159-185), Soukharev & al. (Nucleic Acid Research 1999, vol 27, n° 18, ppE21.1-E21.8), McMinn & al. (Mammalian Genome 2004, vol 15, n° 9, 677-685), Casanova & al (Genesis 2002, vol 32, n° 2, 15/8-160) et Ren & al (Genesis 2002, vol 32, n° 2, 105-108). Cependant, aucun de ces documents ne vient apporter de solution au problème technique associé à la présente invention, ni décrire la solution de la présente invention.

Les vecteurs selon l'invention sont constitués de séquences d'ADN et sont destinés à être insérés dans l'ADN génomique d'une cellule eucaryote. Par séquence d'ADN on entend selon l'invention une molécule d'ADN constituée par une séquence d'acides désoxyribonucléiques liés de manière covalente.

Par cellule eucaryote, on entend plus particulièrement selon l'invention toutes cellules d'origine animale, préférentiellement à l'exception de l'homme, en particulier les cellules de mammifères, préférentiellement de mammifères rongeurs, plus préférentiellement de rat ou de souris.

De manière préférentielle, le vecteur selon l'invention est intégré dans un gène de l'ADN génomique de la cellule eucaryote en aval du promoteur du gène cible, de préférence en amont du premier exon du gène cible.

De manière préférentielle l'introduction des séquences dans l'ADN génomique aboutit à l'inactivation du gène ciblé soit par une stratégie d'insertion soit par une stratégie de délétion de séquence génomique.

Selon un premier mode particulier de réalisation de l'invention le vecteur consiste en les éléments suivants, dans le sens 5'-3':
- un premier site SR1 de reconnaissance d'une recombinase,
- un deuxième site SR2 de reconnaissance d'une recombinase,
- une séquence d'acide nucléique codant pour une séquence d'intérêt et contenant un arrêt de traduction « STOP », cette cassette contenant de manière préférentielle un gène de sélection,
- un troisième site SR3 de reconnaissance d'une recombinase,
- une séquence d'acide nucléique codant pour une séquence d'intérêt et contenant un arrêt de traduction « STOP », l'expression de cette séquence étant placée sous la dépendance du promoteur endogène et ne pouvant avoir lieu sans le retrait de la séquence située entre les sites SR2 et SR3,
- optionnellement une séquence d'acide nucléique d'un gène codant pour un marqueur de sélection fonctionnel dans la cellule eucaryote dans le génome de laquelle il sera intégré,
- un quatrième site SR4 de reconnaissance d'une recombinase.

De manière avantageuse, les sites de reconnaissance de recombinases SR1 et SR2 sont choisis, indépendamment l'un de l'autre, parmi les sites Lox P et FRT et les sites SR1 et SR4 de même que les sites SR2 et SR3 sont deux par deux identiques.

Les recombinases peuvent être délivrées de façon ubiquitaires ou tissue-spécifique. Parmi les moyens permettant une telle délivrance tissus-spécifique, on citera en particulier la délivrance de vecteurs viraux, virus ou rétro-virus, permettant l'expression de la recombinase R2 de manière tissus-spécifique, l'injection des recombinases de manière tissus-spécifique ou encore le croisement des animaux selon l'invention avec des animaux modèles comprenant des éléments permettant l'expression de la recombinase R2 de manière tissus-spécifique.

L'expression de la recombinase R2 de manière tissus-spécifique au moyen de vecteurs viraux peut être réalisée comme suit : Un virus d'expression de la recombinase d'intérêt est préparé et purifié selon les méthodes classiques de préparation virales, bien connues de l'homme du métier. Le virus est alors injecté par stéréotaxie dans la région d'intérêt, par exemple la région cérébrale d'intérêt. La dose de virus ainsi que le nombre d'injection sont des paramètres permettant d'obtenir un nombre de cellules hôtes excisées croissant ainsi qu'une diffusion « géogrpahique » accrue.

L'utilisation de systèmes d'inhalation pour les poumons, d'injection IV pour le foie et les épithéliums et d'injection anaux pour le colon sont d'autres exemples de mode d'administration.

Lorsque l'on dispose d'un modèle animal exprimant la recombinase R2 de manière tissus-spécifique, il est possible d'obtenir un animal exprimant séquence d'intérêt de manière tissus-spécifique par son croisement avec l'animal modèle selon l'invention.

De tels modèles animaux exprimant une recombinase de manière tissus-spécifique sont bien connus de l'homme du métier, décrits notamment dans Xu et al. ((1999). Nat Genet 22, 37-43), Shibata, et al. ((1997). Science 278, 120-3), Kulkarni, et al. ((1999). Cell 96, 329-39), Tsien et al. ((1996). Cell 87, 1327-38) et Harada, et al. ((1999). Embo J 18, 5931-42).

Les combinaisons préférées de sites de reconnaissance sont rappelées dans le tableau 1 ci-dessous.

**Tableau 1**

| SR1 | SR2 | SR3 | SR4 |
|---|---|---|---|
| Lox P | FRT | FRT | LoxP |
| FRT | Lox P | Lox P | FRT |

Dans les deux modes de réalisation, le modèle obtenu après insertion du vecteur selon l'invention dans le génome de la cellule eucaryote, est un modèle KO où le gène endogène est inactivé.

La mise en contact avec une première recombinase reconnue par les sites SR2 et SR3 va permettre d'éliminer l'acide nucléique comprenant une séquence d'arrêt de la transcription, permettant ainsi l'expression de la séquence d'intérêt dans les cellules transformées. Si la recombinase est fournie de façon tissue-spécifique l'expression de la séquence d'intérêt est alors également tissue-spécifique.

La mise ne contact avec la seconde recombinase reconnue par les sites SR1 et SR4 permet d'inactiver le transgène ajouté.

Si l'insertion du vecteur résulte en la délétion de séquences essentielles du gène endogène ou si la seconde recombinase excise des séquences essentielles au gène endogène, le résultat est identique, le gène endogène est délété et le modèle résultant est un KO.

Si l'insertion du vecteur se fait par insertion et ne délète aucune séquence du gène endogène et si la seconde recombinase n'excise aucune séquence essentielle au gène endogène, le résultat est identique, le gène endogène est intact et le modèle retrouve un phénotype normal. Cette action se produit dans toutes les cellules si la recombinase est fournie de façon ubiquitiare. Si la recombinase est fournie de façon tissue-spécifique l'obtention d'un locus délété (KO) ou intact (retour à la normale) est alors également tissue-spécifique.

En fonction des sites de reconnaissance choisis, les recombinases sont choisies, indépendamment l'une de l'autre, parmi les recombinases suivantes : Cre ou FLP.

Les sites de reconnaissance Lox P, Lox P modifié, FRT et les recombinases Cre et FLP correspondantes sont bien connus de l'homme du métier, notamment décrits dans Kilby, et al. ((1993). Trends Genet 9, 413-21), Sauer, et Henderson ((1988). Proc Natl Acad Sci U S A 85, 5166-70), Gu et al. ((1994). Science 265, 103-6), Cohen-Tannoudjiet Babinet ((1998). Mol Hum Reprod 4, 929-38), Shibata, et al. ((1997). Science 278, 120-3), Schlake et Bode ((1994). Biochemistry 33, 12746-51).

Les séquences d'arrêt de la transcription dans les cellules eucaryotes sont bien connues de l'homme du métier. On citera notamment les séquences décrites notamment dans Proudfoot et al (Cell. (1991) 64,:671-4) et dans Beaudoing et al. (Genome Res. (2001) 11, 520-526).

Les gènes codant pour un marqueur de sélection fonctionnel dans les cellules animales sont également bien connus de l'homme du métier. On citera notamment Yagi et al., (1990, PNAS, 87, 9918-22). Les gènes de sélection positive sont bien connus de l'homme du métier et sont préférentiellement des gènes de résistance à un antibiotique, tel les gènes de résistance à la kanamycine, qui permet également la résistance à la néomycine dans les cellules mammifères, résistance à l'hygromycine, à la zéocine, à la blasticidine...

La séquence d'intérêt du vecteur selon l'invention est de préférence choisie parmi les séquences suivantes :
- une séquence d'acide nucléique codant pour un polypeptide, en particulier une protéine d'intérêt, ou un gène rapporteur,
- une séquence d'acide nucléique codant pour un RNA, en particulier un RNA antisens ou un RNAi, miRNA ou siRNA.

De manière avantageuse, la séquence d'acide nucléique codant pour un polypeptide est un ADNc, plus préférentiellement un ADNc codant pour un polypeptide d'origine humaine. On citera notamment ceux décrits dans Li, et al. ((1997). J Cell Biol 139, 129-44), Schneider-Maunoury et al. ((1993). Cell 75, 1199-214) et Tajbakhsh et al. ((1996) Nature 384, 266-70).

De manière avantageuse, le RNA antisens ou interférent permet d'inhiber la traduction d'ARNm cibles, préférentiellement choisis parmi les gènes suivants : GPCR, récepteurs nucléaires, récepteurs membranaires, cytokines, canaux ionique, etc.

La présente invention concerne également un procédé de transformation de cellules eucaryotes pour intégrer dans son génome la combinaison de vecteurs selon l'invention.

Les méthodes d'intégration de séquences dans le génome de cellules eucaryotes sont bien connues de l'homme du métier, notamment décrites dans Smithies et al. ((1985). Nature 317, 230-4) et Wong et al. ((1986). Somat Cell Mol Genet 12, 63-72).

De manière préférentielle, les cellules sont des cellules animales transformées par les méthodes telles que décrites dans Vasquez et al ((2001) PNAS 98, 8403-8410) et Yanez et al ((1999), somatic cell and molecular genetics 25, 27-31).

La présente invention concerne aussi les cellules eucaryotes dans le génome desquelles est intégré la combinaison de vecteurs selon l'invention, plus préférentiellement les cellules animales telles que définies précédemment.

Ces cellules selon l'invention peuvent être des cellules obtenues par intégration de la combinaison de séquences par le procédé selon l'invention. Elles peuvent également comprendre des cellules obtenues par prélèvement sur des animaux obtenus à partir desdites cellules obtenues par le procédé selon l'invention.

L'invention concerne également des embryons d'animaux obtenus à partir des cellules selon l'invention, ainsi que les animaux obtenus à partir de ces embryons et la descendance desdits animaux ayant conservé le vecteur selon l'invention. Les animaux selon l'invention sont définis précédemment, préférentiellement des mammifères à l'exception de l'homme, plus préférentiellement des mammifères rongeurs, encore plus préférentiellement des rats ou des souris.

Les méthodes de préparation d'animaux modèles sont bien connues de l'homme du métier, et notamment décrites pour le mouton (Wilmut et al. Nature 385, 810-813, 1997 ; WO 97 07669), la souris (Wakayama et al. Nature 394, 369-374, 1998 ; WO 99 37143), les bovins (Wells et al. Biol. Reprod. 60, 996-1005, 1999), la chèvre (Baguisi et al. Nature Biotechnol. 17, 456-461, 1999 ; WO 00 25578), le porc (Polejaeva et al. Nature 407, 86-90, 2000), le lapin (Chesne et al. Nature Biotechnol. 20, 366-369, 2002) et le rat (Zhou & al., Science, 25 septembre 2003 ; PCT/FR04/001275). Les méthodes de transfert nucléaire sont notamment décrites par Campbell & al. (Nuclear transfer in practice, School of Biosciences, University of Nottingham, Leicestershire, United Kingdom).

Les animaux modèles ainsi obtenus peuvent être employés de la manière décrite ci-dessous.

Dans une première situation, la présente stratégie permet de générer un animal KO pour un gène d'intérêt. Ce modèle fournit un outil de choix pour analyser la fonction du gène. Dans un second temps, cette approche permet à partir du même animal et par simple croisement avec un animal exprimant la recombinase d'obtenir l'expression du gène Humain correspondant. Ce second modèle permet alors de tester in vivo des modècules régulatrices de cette cible. La qualité du modèle est fortement accrue par l'expression d'une protéine humaine en lieu et place d'une protéine non humaine. En utilisant une seconde recombinase il est alors possible de supprimer l'expression de cette forme Humaine (de façon tissue-spécifique ou ubiquitiare) de façon à vérifier les effets non spécifique des molécules testées in vivo.

Dans une seconde situation, la présente stratégie permet de générer un animal KO pour un gène d'intérêt. Ce modèle fournit un outil de choix pour analyser la fonction du gène. Dans un second temps et en cas de létalité ou de phénotype handicapant la recherche, cette approche permet à partir du même animal et par simple croisement avec un animal exprimant la recombinase de façon tissue-spécifique d'exprimer dans le tissu clef un transgène (le même gène, un gène différent mais permettant de complémenter). Cette expression permet de sauver l'animal ou de corriger certains aspects du phénotype, permettant ainsi une analyse plus poussée des animaux présentant des phénotypes graves. Cette approche est très complémentaire du KO conditionnel, en effet ce dernier inactive un gène dans un tissu d'intérêt (ou quelques tissus d'intérêts) tandis que notre approche permet d'inactiver le gène dans tous les tissus sauf dans un tissu (ou quelques tissus d'intérêts).

## Revendications

1. Vecteur consistant en les éléments suivants, dans le sens 5'-3':
- un premier site SR1 de reconnaissance d'une recombinase,
- un deuxième site SR2 de reconnaissance d'une recombinase,
- une séquence d'acide nucléique codant pour une séquence d'intérêt et contenant un arrêt de traduction « STOP », cette cassette contenant de manière préférentielle un gène de sélection,
- un troisième site SR3 de reconnaissance d'une recombinase,
- une séquence d'acide nucléique codant pour une séquence d'intérêt et contenant un arrêt de traduction « STOP », l'expression de cette séquence étant placée sous la dépendance du promoteur endogène et ne pouvant avoir lieu sans le retrait de la séquence située entre les sites SR2 et SR3,
- optionnellement une séquence d'acide nucléique d'un gène codant pour un marqueur de sélection fonctionnel dans la cellule eucaryote dans le génome de laquelle il sera intégré,
- un quatrième site SR4 de reconnaissance d'une recombinase.

2. Vecteur selon la revendication 1, **caractérisé en ce que** les sites de reconnaissance SR1 et SR2 et SR3 et SR4 sont identiques deux par deux.

3. Vecteur selon la revendication 2, **caractérisé en ce que** les sites SR1 et SR2 sont différents des sites SR3 et SR4.

4. Vecteur selon l'une des revendications 1 à 3, **caractérisé en ce que** les sites de reconnaissance de recombinases SR1, SR2, SR3 et SR4 sont choisis, indépendamment l'un de l'autre, parmi les sites Lox P, Lox P modifiés et FRT.

5. Cellules eucaryotes à l'exception de l'homme dans le génome desquelles est intégré un vecteur selon l'une des revendications 1 à 4.

6. Cellules selon la revendication 5, **caractérisées en ce qu'**elles sont choisies parmi les cellules de mammifères, à l'exception de l'homme, préférentiellement de mammifères rongeurs, de préférence rat ou souris.

7. Embryons d'animaux, à l'exception de l'homme, obtenus à partir des cellules selon l'une des revendications 5 ou 6.

8. Animaux, à l'exception de l'homme, obtenus à partir des embryons selon la revendication 7, comprenant des cellules selon l'une des revendications 5 ou 6.

## Claims

1. A vector consisting in the 5'-3' sense, in the following elements:
- a first recombinase target site SR1,
- a second recombinase target site SR2,
- a nucleic acid sequence coding for a sequence of interest and containing a translation signal "STOP", this cassette preferentially containing a selection gene,
- a third recombinase target site SR3,
- a nucleic acid sequence coding for a sequence of interest and containing a translation signal "STOP", the expression of this sequence being placed under the dependency of the endogenous promoter and which cannot occur without removing the sequence located between the sites SR2 and SR3,
- optionally, a nucleic acid sequence of a gene coding for a functional selection marker in the eukaryotic cell in the genome of which it will be integrated,
- a fourth recombinase target site SR4.

2. The vector according to claim 1, **characterized in that** the recombinase target sites SR1 and SR2 and SR3 and SR4 are identical two by two.

3. The vector according to claim 2, **characterized in that** the SR1 and SR2 sites are different from the SR3 and SR4 sites.

4. The vector according to any of claims 1 to 3, **characterized in that** the recombinase target sites SR1, SR2, SR3, SR4 are selected independently of each other, from the sites Lox P, modified Lox P and FRT.

5. Eukaryotic cells in the genome of which a vector according to any of claims 1 to 4 is integrated.

6. The cells according to claim 5, **characterized in that** they are selected from cells of mammals, excluding humans, preferentially from rodent mammals, preferably from rats or mice.

7. Embryos of animals, excluding humans, obtained from cells according to any of claims 5 or 6.

8. Animals, excluding humans, obtained from embryos according to claim 7, comprising cells according to any of claims 5 or 6.

## Patentansprüche

1. Vektor, welcher in 5'-3'-Richtung aus folgenden Elementen besteht:
- einer ersten Rekombinase-Erkennungsstelle SR1,
- einer zweiten Rekombinase-Erkennungsstelle SR2,
- einer Nukleinsäuresequenz, welche für eine Sequenz von Interesse kodiert und einen Translations-Stopp «STOP» enthält, wobei diese Kassette bevorzugt ein Selektionsgen enthält,
- einer dritten Rekombinase-Erkennungsstelle SR3,
- einer Nukleinsäuresequenz, welche für eine Sequenz von Interesse kodiert und einen Translations-Stopp «STOP» enthält, wobei die Expression dieser Sequenz unter der Kontrolle des endogenen Promotors steht und ohne das Verschwinden der zwischen den Stellen SR2 und SR3 liegenden Sequenz nicht stattfinden kann,
- optional einer Nukleinsäuresequenz eines Gens, welches für einen Selektionsmarker kodiert, der in der eukaryotischen Zelle, in dessen Genom das Gen integriert wird, funktionell ist,
- einer vierte Rekombinase-Erkennungsstelle SR4.

2. Vektor gemäß Anspruch 1, **dadurch gekennzeichnet, dass** jeweils zwei der Erkennungsstellen SR1 und SR2 und SR3 und SR4 identisch sind.

3. Vektor gemäß Anspruch 2, **dadurch gekennzeichnet, dass** sich die Stellen SR1 und SR2 von den Stellen SR3 und SR4 unterscheiden.

4. Vektor gemäß einem der Ansprüche 1 bis 3, **dadurch gekennzeichnet, dass** die Rekombinase-Erkennungsstellen SR1, SR2, SR3 und SR4 unabhängig voneinander aus Lox P-, modifizierten Lox P- und FRT-Stellen ausgewählt sind.

5. Eukaryotische Zellen, ausgenommen des Menschen, in deren Genom ein Vektor gemäß einem der Ansprüche 1 bis 4 integriert ist.

6. Eukaryotische Zellen gemäß Anspruch 5, **dadurch gekennzeichnet, dass** sie aus Zellen von Säugetieren, ausgenommen des Menschen, bevorzugt Nagetieren, bevorzugt Ratten oder Mäusen, ausgewählt sind.

7. Embryos von Tieren, ausgenommen des Menschen, die ausgehend von Zellen gemäß einem der Ansprüche 5 oder 6 erhalten werden.

8. Tiere, ausgenommen Menschen, die ausgehend von Embryos gemäß Anspruch 7 erhalten werden und Zellen gemäß einem der Ansprüche 5 oder 6 enthalten.
